# EUROPÄISCHE PATENTSCHRIFT

(11) **EP 1 658 040 B1**
(45) Veröffentlichungstag und Bekanntmachung des Hinweises auf die Patenterteilung: **18.03.2009**
(21) Anmeldenummer: 04764510.6
(22) Anmeldetag: 26.08.2004
(51) Int. Cl.: A61K 8/18, A61K 33/38

(54) **PRODUKT MIT SILBER UND ZINK**
SILVER-AND ZINC-CONTAINING PRODUCT
PRODUIT COMPRENANT DE L'ARGENT ET DU ZINC

(30) Priorität: 29.08.2003 DE 10340276
(43) Veröffentlichungstag der Anmeldung: 24.05.2006
(60) Teilanmeldung: 07020782.4
(73) Patentinhaber: Bio-Gate AG, 90411 Nürnberg (DE)
(72) Erfinder: BECHERT, Thorsten, 91301 Forchheim (DE); WAGENER, Michael, 28355 Bremen (DE); STEINRÜCKE, Peter, 91052 Erlangen (DE)
(74) Vertreter: Ehnis, Tobias
(86) Internationale Anmeldenummer: PCT/EP2004/009534
(87) Internationale Veröffentlichungsnummer: WO 2005/023206

(56) Entgegenhaltungen:
- WO-A-00/06208
- WO-A-00/78281
- WO-A-02/17984
- US-A- 4 828 832
- US-A- 5 744 151
- US-A1- 2002 001 628
- PATENT ABSTRACTS OF JAPAN Bd. 2002, Nr. 10, 10. Oktober 2002 (2002-10-10) & JP 2002 179514 A (NISSHIN STEEL CO LTD), 26. Juni 2002 (2002-06-26)

## Beschreibung

Die Erfindung betrifft ein Körperpflegemittel, ein Verfahren zur Herstellung eines solchen Körperpflegemittels sowie eine Verwendung zur Herstellung eines Medikaments zur Behandlung einer Entzündung und/oder Infektion.

Aus der JP 11060417 A sind anorganische Oxid-Pulver bekannt, welche in Kosmetika verwendet werden können. Die Pulverpartikel weisen eine Größe von unter 1 µm auf und sind auf ihren Oberflächen mit mehreren Oxiden von beispielsweise Zink oder Silber beschichtet. Die Partikel können mit einer Silber-Zink-Legierung von 20 bis 80% w/w (Gewichtsprozent) Silber und 80 bis 20% w/w Zink beschichtet werden, wobei das Gewicht der Beschichtung 0,1 bis 10% w/w beträgt. Die Partikel werden nach der Beschichtung in Luft für ungefähr eine Stunde auf 300 bis 400°C erhitzt. Es ist anzunehmen, dass durch diese Behandlung die Silber-Zink-Beschichtung zumindest teilweise in Form von Oxiden vorliegt. Die in den Partikeln enthaltenen Silber-Ionen liegen zu einem großen Teil in Form von Silberoxid vor. Die Abgabe von Silber-Ionen durch diese Partikel hängt sehr von der chemischen und biologischen Umgebung ab. Beispielsweise kann das Silberoxid in entsprechender biologischer Umgebung in Silbersulfid umgewandelt werden. Silber-Ionen können dann nicht mehr freigesetzt werden.

Aus der DE 39 32 469 C2 und der JP 04170960 A ist Hydroxyapatit bekannt, welches adsorbierte Silber- und Zink-Ionen enthält. Gemäß der JP 04170960 A ist der Anteil an Zink mindestens 5% w/w gegenüber dem Silber. Der wesentliche Nachteil der Verwendung von Hydroxyapatit als Träger für Silber- und Zink-Ionen ist, dass Hydroxyapatit wie ein Ionentauscher wirkt. Das führt dazu, dass die daran gebundenen Ionen in Abhängigkeit von der Ionenkonzentration in der Umgebung freigesetzt werden. Die Freisetzung der Ionen ist daher schlecht zu kontrollieren und in einem Körperpflegemittel, welches wechselnden Ionenkonzentrationen in der Umgebung ausgesetzt ist, nicht konstant.

Aus der WO 00/06208 A1 ist eine Zahnpasta bekannt, die antimikrobielle Keramikpartikel bzw. Zeolith enthält, worin ein Teil der austauschbaren Ionen durch antimikrobiell wirksame Silber- und Zink-Ionen ersetzt ist. Auch diese Partikel wirken wie Ionentauscher und weisen die oben genannten Nachteile auf.

Aus der DE 101 41 117 A1 und der US 6,143,318 A sind Gläser als Träger für Zink- und Silber-Ionen bekannt. Diese Gläser setzen die Zink- und Silber-Ionen nach Art eines Ionentauschers frei. Sie weisen daher ebenfalls die oben genannten Nachteile auf.

Aus der WO 02/17984 A1 ist ein antimikrobielles Material zum Implantieren in Knochen oder zum Beschichten oder Herstellen eines Implantats oder einer implantierbaren medizinischen Vorrichtung bekannt. Dabei sind aus einem antimikrobiellen Metall gebildete Partikel in einem im ausgehärteten Zustand eine Matrix bildenden Matrixmaterial fein verteilt. Das Metall kann aus einem oder mehreren der folgenden Bestandteile gebildet sein: Ag, Au, Pt, Pd, Ir, Sn, Cu, Sb, Zn.

Die US 5,744,151 A offenbart eine pharmazeutische Zusammensetzung, welche eine Silberionen enthaltende Verbindung enthält.

Die US 2002/0001628 A1 offenbart eine pharmazeutische Zusammensetzung zum Verhindern oder Heilen von Verdauungsstörungen. Die Zusammensetzung enthält Silber- und Zinkionen enthaltende Aluminosilikate.

Aus der WO 00/78281 A1 ist ein antimikrobielles Körperpflegemittel bekannt, welches in einem menschliche oder tierische Haut und/oder Mukosa kontaktierenden Teil eine organische Matrix aufweist. Diese Matrix enthält homogen dispergierte Partikel von metallischem Silber. Die Partikel haben dabei eine Größe zwischen 1 und 50 nm und sind in einer Menge enthalten, welche auf der Oberfläche des die Haut und/oder Mukosa kontaktierenden Teils eine antimikrobiell wirksame aber weniger als zytotoxische Konzentration bereitstellt. Bei dem Körperpflegemittel kann es sich beispielsweise um eine Salbe oder eine Creme handeln.

Aufgabe der vorliegenden Erfindung ist es, ein alternatives Körperpflegemittel mit verbesserter Wirksamkeit bereitzustellen. Insbesondere soll das Körperpflegemittel über einen langen Zeitraum verhältnismäßig konstant antimikrobiell und gegebenenfalls auch antiinflammatorisch wirken.

Diese Aufgabe wird durch die Merkmale der Patentansprüche 1, 22 und 25 gelöst. Zweckmäßige Ausgestaltungen ergeben sich aus den Merkmalen der Patentansprüche 2 bis 21, 23, 24 und 26 bis 45.

Erfindungsgemäß ist ein Körperpflegemittel vorgesehen, welches metallische Partikel enthält, von denen im Körperpflegemittel oder bei einem Kontakt mit Körperflüssigkeit oder Körperfeuchtigkeit Zink-Ionen und Silber-Ionen freigesetzt werden. Die Zink-Ionen und Silber-Ionen müssen dabei nicht notwendigerweise jeweils von ein und demselben Partikel freigesetzt werden. Die genannten Ionen wirken bereits in geringsten Konzentrationen antimikrobiell. Metallische Partikel, d. h. Partikel, welche aus Metall bestehen, können über einen langen Zeitraum verhältnismäßig konstant eine niedrige Menge an Zink-Ionen und Silber-Ionen freisetzen. Dadurch kann eine Entstehung einer möglicherweise schädlichen Konzentration an Zink- oder Silber-Ionen in der Haut und/oder Mukosa vermieden werden. Die Partikel weisen einen Gehalt an metallischem Silber von mindestens 99% w/w (Gewichtsprozent) auf. Bei einem solch hohen Silbergehalt kommt ein zytotoxischer Effekt anderer Metall-Ionen, insbesondere von Kupfer-Ionen, nicht zum Tragen. Die den Metallgehalt angebenden Prozentangaben beziehen sich hier und im Folgenden wenn nicht anders angegeben auf den Gewichtsanteil der angegebenen Metalle am Gesamtgewicht der Partikel. Es sind Angaben von Gewichtsprozenten (% w/w).

Zum Erreichen der antimikrobiellen und gegebenenfalls auch antiinflammatorischen Wirkung hat es sich als vorteilhaft erwiesen, wenn der Gehalt an metallischem Silber mindestens 99% w/w und demnach der Gehalt an metallischem Zink höchstens 1% w/w beträgt. Eine antiinflammatorische Wirkung kann erzielt werden, wenn die Partikel in einer höheren Konzentration in dem Körperpflegemittel enthalten sind als zur Erreichung einer bloß antimikrobiellen Wirkung erforderlich ist. Die Partikel stellen ein Depot für Zink- und Silber-Ionen dar, welches diese Ionen unter üblichen Einsatzbedingungen eines Körperpflegemittels über einen langen Zeitraum abgeben kann. Darüber hinaus erlauben es metallische Partikel, die von den Partikeln gemäß der JP 11060417 A bereitgestellte Menge an Silber-Ionen bei einer wesentlich geringeren Partikelgröße bereitzustellen. Durch die geringere Größe der Partikel sind diese sedimentationsstabiler und lassen sich besser in Körperpflegemittel, wie beispielsweise Öle oder Salben, einmischen. Da Körperpflegemittel oft über lange Zeiträume in ihrer Zusammensetzung stabil sein müssen, ist dies ein wesentlicher Vorteil der im erfindungsgemäßen Körperpflegemittel eingesetzten Partikel.

Körperpflegemittel sind Produkte, welche mit der menschlichen oder tierischen Haut und/oder Mukosa in Kontakt gebracht werden, um eine reinigende, schützende, therapeutische, heilende, pflegende, kosmetische oder lindernde Wirkung zu erzielen. Die Körperpflegemittel können aus einem Naturstoff und davon abgeleiteten Derivaten hergestellt sein oder diese Naturstoffe enthalten. Sie können auch aus Kunststoffen hergestellt sein oder Kunststoffe enthalten, welche die Partikel enthalten. Die Kunststoffe können z. B. sein: Polyethylene und daraus abgeleitete Copolymere, Polypropylene und daraus hergestellte Polyblends, Polybutene, Polystyrole in Homo- und Copolymerisaten, Acryl-Butadien-Styrol-Terpolymerisat (ABS), Synthesekautschuks, Hart- und Weich-PVC, Polytetrafluorethen (PTFE), Polychlortrifluorethylen (PCTFE) und andere Fluorpolymere, Polyvinylether, Polyvinylacetate, Polyvinylpropionate, Polyvinylalkohole, Copolymere des Vinylalkohols, Polyvinylacetale, Polyethylenglykole, Acrylpolymerisate, Polymetacrylsäuremethylester, Polyacrylnitril, Polycyanoacrylate, Polymere auf Polymethacrylimidbasis, Polyacrylimide, Polyvinylamine, Polyamide einschließlich Polyphenylenisophtalamid, Poly(p-phenylenterephtalamid), lineare Polyurethane und Polyester einschließlich Polyethylenterephthalat (PET), Polybutylen- terephthalat (PBT) und Polytetramethylenterephthalat (PTMT), Polycarbonate und daraus abgeleitete Polymere, Polyoxymethylene (POM), Polyether, Polyetheretherketone, Polyetherblockamide, Kondensationsharze, wie Phenolplaste und Aminoplaste, vernetzte Polyester einschließlich Polyesterharze, Epoxyharze, vernetzte Polyurethane, Reaktionsharze auf Methylmethacrylat-Basis, Polysiloxane und andere Polymere mit anorganischer Hauptkette.

Die Körperpflegemittel können auch, insbesondere medizinisch wirksame, Präparate sein, wie Emulsionen, Lotionen, Gele, Cremes, Salben, Heilsalben, Puder, Kosmetika, Hautschutzcremes oder -salben, Desinfektionsmittel oder antiinflammatorische Heilmittel, Suspensionen, Seifen, synthetische Tenside, Badezusätze, Peelingpräparate, Gesichtswässer, Zahnpflegemittel, Zahncremes, Mundwässer, Zahnreinigungskaugummis, Prothesenhaftmittel, Haarshampoos, Sonnenschutzmittel, etc.. Diese Produkte enthalten häufig entweder ein Polymer oder einen organischen Bestandteil in einem Träger, welcher ein gutes Substrat für eine Vielzahl von Mikroorganismen sein kann. Ein Wachstum dieser Mikroorganismen in diesen Substraten kann hygienische oder medizinische Probleme verursachen.

Die Partikel können in dem Körperpflegemittel in einer Menge enthalten sein, die an einer Stelle des Kontakts des Körperpflegemittels mit der Haut und/oder Mukosa eine antimikrobiell wirksame aber weniger als zytotoxische Konzentration an Zink-Ionen und Silber-Ionen ermöglicht.

Die Partikel können in dem Körperpflegemittel so vorliegen, dass erst beim Kontakt mit Körperfeuchtigkeit der Haut und/oder Mukosa Zink- oder Silber-Ionen freigesetzt werden. Das kann beispielsweise der Fall sein, wenn die Partikel in einer öligen oder fettigen Zubereitung vorliegen, in der sie keinen Kontakt zu Wasser haben oder wenn sie in einem trockenen Körperpflegemittel enthalten sind. Körperflüssigkeit oder Körperfeuchtigkeit kann z. B. Hautfeuchtigkeit, Blut, Schweiß, Lymphflüssigkeit, Menstruationsflüssigkeit oder Urin sein. Die Ionen können im Körperpflegemittel auch unabhängig von einem Kontakt mit Körperflüssigkeit oder Körperfeuchtigkeit freigesetzt werden. Das kann z. B. der Fall sein, wenn das Körperpflegemittel ein Gel oder eine Creme ist und die Partikel die Ionen an eine darin enthaltene wässrige Phase abgeben.

Das erfindungsgemäße Körperpflegemittel wirkt antimikrobiell und gegebenenfalls auch antiinflammatorisch. Darüber hinaus benötigt es wegen der antimikrobiellen Wirkung der Metall-Ionen neben den Partikeln keine Konservierungsstoffe. Es kann als, insbesondere medizinische/s, Heil- oder Pflegesalbe,-creme oder -gel ausgebildet sein. Ein solches Präparat kann wegen der entzündungshemmenden Wirkung alternativ zu Kortikoid enthaltenden Präparaten medizinisch angewandt werden. Als Handsalbe, -creme oder -gel schützt die antimikrobielle Wirkung auch vor dem Übertragen von Krankheitserregern, z. B. durch Händeschütteln, und verhindert bei kleinen Wunden an den Händen das Eindringen von Keimen. Dadurch, dass auf Konservierungsstoffe verzichtet werden kann, treten darüber hinaus weniger, insbesondere allergische, Unverträglichkeitsreaktionen auf. Dadurch, dass von den Partikeln neben den antimikrobiell wirkenden Silber-Ionen ebenfalls antimikrobiell wirkende Zink-Ionen freigesetzt werden, wird ein besonders effektives Körperpflegemittel bereitgestellt. Die Zink-Ionen und die Silber-Ionen unterstützen sich gegenseitig in der antimikrobiellen Wirkung. Das liegt unter anderem daran, dass sie in ihrer antimikrobiellen Wirkung eine unterschiedliche Spezifität für Mikroorganismen aufweisen. Darüber hinaus weist Zink in Kombination mit Silber eine besonders gute wundheilende und entzündungshemmende Wirkung auf. Ursache dafür könnte sein, dass durch das Silber das Wachstum von die Wundheilung störenden Mikroorganismen unterbunden wird, deren Wachstum durch Zink-Ionen alleine nicht gehemmt wird. Das erfindungsgemäße Körperpflegemittel ist insbesondere für Patienten geeignet, die dauerhaft eine gesteigerte Sorgfalt auf Körperpflege und Körperhygiene verwenden müssen. Das können z.B. Personen sein, die ein geschwächtes Immunsystem und/oder ein erhöhtes Risiko haben, an Hautinfektionen zu erkranken, wie beispielsweise Diabetiker.

Vorteilhaft ist es für die Körperpflege, wenn von den Partikeln in einer definierten Zeiteinheit mehr Silber-Ionen als Zink-Ionen freigesetzt werden. Die definierte Zeiteinheit kann z. B. die Zeit während eines Kontakts des Körperpflegemittels mit Haut und/oder Mukosa oder des Kontakts mit der Körperflüssigkeit oder Körperfeuchtigkeit sein. Die definierte Zeiteinheit kann z. B. eine, 4, 8 oder 12 Stunden sein. Vorzugsweise können von den Partikeln im Körperpflegemittel oder bei einem Kontakt mit Körperflüssigkeit oder Körperfeuchtigkeit auch Kupfer-Ionen freigesetzt werden. Auch Kupfer-Ionen weisen eine antimikrobielle Wirkung auf, wobei sich das wirkungsspektrum von dem der Zink-Ionen und Silber-Ionen unterscheidet. Dadurch kann eine noch bessere wundheilende und entzündungshemmende Wirkung erzielt werden als mit der Kombination von Silber- und Zink-Ionen. Bevorzugt werden von den Partikeln während der Zeiteinheit mehr Silber-Ionen als Kupfer-Ionen freigesetzt. Weiterhin ist es vorteilhaft, wenn während der Zeiteinheit mehr Zink-Ionen als Kupfer-Ionen freigesetzt werden. Um mehr Ionen eines ersten Metalls (z. B. Silber) als Ionen eines zweiten Metalls (z. B. Zink) freizusetzen, können die Partikel bspw. vom ersten Metall eine größere Menge beinhalten als vom zweiten Metall.

Die Partikel weisen vorzugsweise einen Gehalt an metallischem Silber von mindestens 99,5% w/w auf. Durch einen solchen Silbergehalt wird das Körperpflegemittel noch verträglicher, weil Nebenwirkungen anderer Metall-Ionen, insbesondere ein zytotoxischer Effekt, z. B. von Kupfer-Ionen, oder die Auslösung von Allergien, nahezu nicht stattfinden.

Die Partikel können bis zu 0,5% w/w metallisches Zink enthalten. Auch metallisches Kupfer kann bis zu einem Gehalt von 0,5% w/w enthalten sein. Bevorzugt sind die Partikel aus einer Silber-Zink-Legierung oder einer Silber-Zink-Kupfer-Legierung gebildet. Vorzugsweise weisen die Partikel Verunreinigungen von weniger als 5 ppm an Kalium, Natrium oder Chlor auf. Größere Verunreinigungen des Silbers können unerwünschte Nebenwirkungen auslösen.

Die in dem Körperpflegemittel enthaltenen Partikel weisen vorzugsweise einen Durchmesser von 1 bis 50 nm, insbesondere 5 bis 15 nm, vorzugsweise 10 nm, auf. Das ermöglicht es, ein Körperpflegemittel, wie eine Salbe oder eine Creme bereitzustellen, dass keine Hilfsmittel zum Dispergieren der Partikel aufweist. Weiterhin ist festgestellt worden, dass die Metall-Ionen eine konservierende Wirkung aufweisen. Daher kann neben den Partikeln auf Konservierungsstoffe verzichtet werden. Unerwünschte, insbesondere allergische, von einem Dispergierhilfsmittel oder einem üblichen Konservierungsstoff, wie z. B. Formaldehyd, ausgelöste Reaktionen können dadurch vermieden werden.

Die Partikel können zumindest teilweise auch metallisches Silber enthaltende poröse Partikel mit einem mittleren Durchmesser zwischen 1 und 100 µm sein. Die mittlere innere Porosität der Partikel kann mindestens 65%, insbesondere zwischen 65 und 95%, bevorzugt zwischen 65 und 90%, insbesondere zwischen 70 und 85%, vorzugsweise zwischen 75 und 85%, oder bevorzugt zwischen 85 und 95%, insbesondere zwischen 90 und 95%, betragen. Die Partikel können durch ihre Größe von 1 bis 100 µm beim bestimmungsgemäßen Gebrauch des Körperpflegemittels kaum oder gar nicht von außen, z. B. durch die Haut, in die Blutbahn eines Menschen oder Säugetiers eindringen. Der antimikrobielle Effekt ist allein auf die Hautoberfläche beschränkt. Die Auslösung von Allergien und unerwünschten toxischen Effekten wird dadurch vermieden. Gleichzeitig gewährleistet die Porosität der Partikel, dass eine antimikrobiell und gegebenenfalls auch antiinflammatorisch wirksame Konzentration an Silber-, Zink- und ggf. Kupfer-Ionen durch die Partikel bereitgestellt werden kann. Diese Ionen wirken vor allem auf der Oberfläche der das Körperpflegemittel kontaktierenden Haut- bzw. Schleimhaut und haben keinen negativen Einfluss auf darunter liegendes Gewebe. Die Partikel sind dadurch und wegen ihrer ein Eindringen in die Haut verhindernden Größe sehr hautverträglich und biokompatibel. Das Körperpflegemittel ist dadurch insbesondere für Patienten geeignet, die dauerhaft eine gesteigerte Sorgfalt auf Körperpflege und Körperhygiene verwenden müssen, wie beispielsweise Diabetiker.

Unter innerer Porosität wird der prozentuale Anteil des Volumens des Partikel verstanden, der nicht von Metall ausgefüllt ist. Die mittlere innere Porosität der Partikel kann nach folgendem Verfahren bestimmt werden:
1. Einbetten der Partikel in einen Kunststoff,
2. Herstellung von Ultradünnschnitten der eingebetteten Partikel,
3. Anfertigen von Transmissions-Elektronenmikroskop(TEM)-Aufnahmen der Partikel,
4. Bestimmung des prozentualen Anteils der nicht von Metall ausgefüllten Fläche jeweils innerhalb eines Partikel im Verhältnis zur Gesamtfläche dieses Partikels in einer Mehrzahl der TEM-Aufnahmen und
5. Berechnen des Mittelwerts einer Mehrzahl so bestimmter prozentualer Anteile.

Der Schritt Ziff. 4 kann dabei durch eine computergestützte Bildauswertung der TEM-Aufnahmen erfolgen. Neben der inneren Porosität kann auch die Gesamtporosität der Partikel bestimmt werden. Dazu wird zunächst die Klopfdichte eines Pulvers der Partikel bestimmt. Die Klopfdichte ist die Masse einer Volumeneinheit eines durch Klopfen möglichst dicht gelagerten Pulvers. Die Klopfdichte kann nach DIN ISO 3953 bestimmt werden. Der dabei ermittelte Wert wird als prozentualer Anteil an der Dichte des die Partikel bildenden Metalls, hier Silber mit einer Dichte von 10,49 g/cm³, berechnet und von 100% subtrahiert. Der so berechnete Wert stellt die Gesamtporosität der Partikel dar. Er kann für die in dem erfindungsgemäßen Körperpflegemittel enthaltenen Partikel zwischen 85 und 95%, insbesondere zwischen 90 und 95%, vorzugsweise zwischen 93 und 95% liegen.

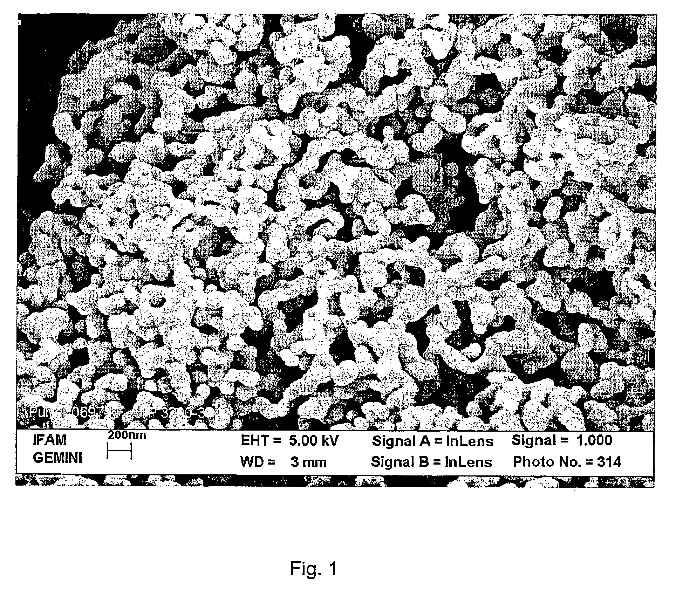

Die porösen Partikel sind besonders gut zu einer lang anhaltenden verhältnismäßig konstanten Abgabe der Silber- und Zink-Ionen geeignet, wenn sie als Agglomerate metallischer Primärpartikel vorliegen. Die Agglomerate können durch thermisches Verdampfen des die Agglomerate bildenden Metalls und anschließendem Abscheiden des Metalldampfs auf einem Metallfilter erzeugt werden. Vorzugsweise sind die Agglomerate aus Primärpartikeln mit einem mittleren Durchmesser zwischen 10 und 200 nm, vorzugsweise zwischen 15 und 80 nm, gebildet. Primärpartikel dieser Größe erlauben eine ausreichende Freisetzung von Silber-Ionen und sind gut herzustellen. DiePrimärpartikel lassen sich auf Grund ihrer äußeren Form und Größe elektronenmikroskopisch identifizieren. Sie sind bspw. in Fig. 1 als kugelige Gebilde zu erkennen. Die Primärpartikel sind miteinander über Sinterhälse verbunden. Der mittlere Abstand zwischen den jeweils äußersten Primärpartikeln an der Oberfläche der Agglomerate liegt bevorzugt im Bereich von 20 bis 200 nm, vorzugsweise 100 bis 200 nm.

Die porösen Partikel weisen vorzugsweise eine schwammartige Struktur auf. Durch die dadurch bereitgestellte große Oberfläche können Silber-, Zink- und gegebenenfalls Kupfer-Ionen in ausreichender Menge freigesetzt werden, um antimikrobiell und gegebenenfalls auch antiinflammatorisch wirksam zu sein.

Bevorzugt weisen die Partikel einen mittleren Außendurchmesser von 2 bis 20 µm, vorzugsweise 2 bis 5 µm, auf. Die spezifische Oberfläche der Partikel kann zwischen 2 und 10 m²/g, insbesondere zwischen 3 und 6 m²/g, vorzugsweise zwischen 3,5 und 4,5 m²/g, liegen. Die spezifische Oberfläche kann z. B. mittels N₂ - Adsorption volumetrisch nach der BET-Methode bestimmt werden. Die BET-Methode ist eine nach Brunauer, Emmett und Teller benannte Methode zur Bestimmung der Oberfläche und gegebenenfalls auch der Poren-Größenverteilung von festen Körpern (z. B. Pulvern), die davon ausgeht, dass Gase, Dämpfe etc. auf festen Körpern unter Freisetzung einer messbaren Adsorptionswärme zunächst in einer monomolekularen Schicht adsorbiert werden. Beispielsweise kann das Volumen an Stickstoff-Gas, das bei -196°C in Abhängigkeit vom angewandten Druck auf dem Adsorptionsmittel adsorbiert wird, gemessen werden.

Die Partikel können in einem Trägermaterial enthalten sein, welches aus einem Silikonöl, einem Mineralöl, Glyzerin oder einem üblichen aus der Pharmakologie bekannten Salbenbestandteil besteht. Zur Herstellung eines erfindungsgemäßen Körperpflegemittels können die Partikel durch einen Sputterprozess hergestellt werden, bei dem das die Partikel bildende Metall verdampft wird und der metallische Dampf direkt in eine Trägerflüssigkeit abgeschieden wird, die dann in das Körperpflegemittel eingebracht wird. Die Besonderheit des Verfahrens besteht darin, dass das verdampfte Metall nicht auf einem festen Träger, wie z. B. Puderpartikel, sondern in eine Flüssigkeit hinein abgeschieden wird, welche direkt in das Körperpflegemittel eingearbeitet werden kann. Die Struktur der dabei entstehenden Silber-Zink-Partikel und die mittels dieses Verfahrens erzielbaren Partikelgrößen sind besonders vorteilhaft für das Einbringen dieser Partikel in Körperpflegemittel. Die Trägerflüssigkeit kann ein Silikonöl, ein Mineralöl, Glyzerin oder ein üblicher aus der Pharmakologie bekannter Salbenbestandteil sein. Auch die Agglomerate können in eine solche Trägerflüssigkeit aufgenommen werden, die in das Körperpflegemittel eingebracht wird. Vorteilhafterweise weist die Trägerflüssigkeit bei 20 °C einen Dampfdruck von weniger als 250 mbar, insbesondere weniger als 70 mbar, vorzugsweise weniger als 10 mbar, insbesondere weniger als 3 mbar, auf.

Darüber hinaus betrifft die Erfindung die Verwendung von metallischen Partikeln zur Herstellung eines Medikaments zur Behandlung einer Entzündung und/oder Infektion bei einem Säugetier oder Menschen, wobei von den Partikeln im Medikament oder bei einem Kontakt mit Körperflüssigkeit oder Körperfeuchtigkeit Zink-Ionen und Silber-Ionen freigesetzt werden. Die Partikel weisen einen Gehalt an metallischem Silber von mindestens 99% w/w auf. Übliche Medikamente zur Behandlung einer Entzündung bei einem Säugetier oder Menschen weisen oft eine Kombination von antiinflammatorischen und antimikrobiellen Wirkstoffen auf. Der antimikrobielle Wirkstoff soll eine Infektion, insbesondere mit Staphylococcus aureus, verhindern oder bekämpfen. Üblicherweise handelt es sich bei dem antimikrobiellen Wirkstoff um ein Antibiotikum. Alternativ kann das Antibiotikum auch systemisch verabreicht werden, während der antiinflammatorische Wirkstoff lokal, z. B. topisch, verabreicht wird. Wegen der, insbesondere bei langfristiger Anwendung, bestehenden Gefahr der Entstehung von Antibiotika-Resistenzen sollte der Einsatz von Antibiotika jedoch auf ein Mindestmaß reduziert werden. Als antiinflammatorischer Wirkstoff wurde bisher z. B. ein Kortikoid wie Kortison verwendet, das jedoch eine große Zahl von Nebenwirkungen aufweist. Der wesentliche Vorteil des erfindungsgemäß hergestellten Medikaments besteht darin, dass die Partikel sowohl eine antiinflammatorische als auch eine antimikrobielle Wirkung aufweisen. Der Einsatz von Antibiotika kann reduziert und die Nebenwirkungen der Kortikoide oder anderer antiinflammatorischer Wirkstoffe können vermieden werden.

Die Behandlung erfolgt topisch, d. h. beispielsweise durch Auftragen auf die Haut oder eine Wunde. Das Medikament kann eine Salbe, eine Creme oder ein Gel sein. Weitere vorteilhafte Ausgestaltungen der Verwendung ergeben sich aus den vorangehenden, das erfindungsgemäße Körperpflegemittel betreffenden Ausführungen.

Nachfolgend wird die Erfindung anhand von Ausführungsbeispielen näher erläutert. Es zeigen:
- Fig. 1: eine rasterelektronenmikroskopische Aufnahme eines Silber-Agglomerats und
- Fig. 2: eine Matrix grafischer Darstellungen des zeitlichen Verlaufs des in Form von optischer Dichte (OD) eines Mediums gemessenen Wachstums von Bakterien in Kontakt mit verschiedenen cremeförmigen Körperpflegemitteln.

Fig. 1 zeigt eine rasterelektronenmikroskopische Aufnahme eines Silber-Agglomerats. Das Silber-Agglomerat besteht hier im Wesentlichen aus kugeligen Primärpartikeln mit einer mittleren Korngröße von etwa 60 nm. Die Primärpartikel sind im Wesentlichen über Sinterhälse miteinander verbunden. Sie bilden ein hochporöses Gerüst. Das hier gezeigte Silber-Agglomerat hat eine Größe von etwa 10 µm.

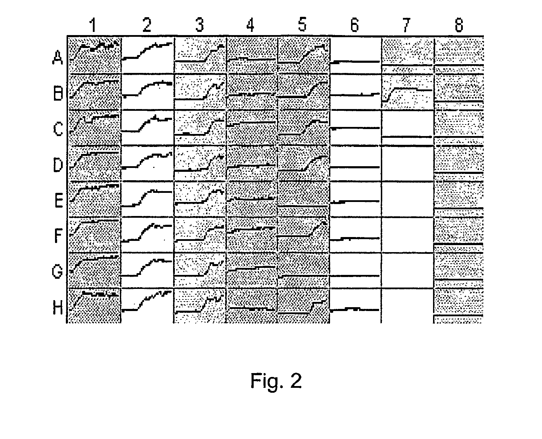

Die in Fig. 2 gezeigten Ergebnisse sind nach dem aus der DE 197 51 581 A1 bekannten Verfahren ermittelt worden. Dieses Verfahren ist ferner beschrieben in Bechert, Thorsten et al., Nature Medicine (2000), Bd. 6, Nr. 8, Seiten 1053 bis 1056. Der Offenbarungsgehalt der beiden vorgenannten Dokumente wird hier einbezogen. Die zu testenden erfindungsgemäßen Körperpflegemittel wurden in Form von Cremes hergestellt, jeweils auf einem Werkstoff als Träger aufgetragen und in dem Test wie beschrieben eingesetzt. Im Einzelnen wurde der Test wie folgt durchgeführt:

Es werden zunächst verschiedene Cremeproben hergestellt. Auf jeden Träger wird eine Menge von 11 mg der jeweiligen Creme aufgetragen. Anschließend werden in jede Vertiefung einer Mikrotiterplatte 200 µl einer Staphylococcus epidermidis enthaltenden Lösung gefüllt. Die Träger mit den Cremeproben werden jeweils in einer der Vertiefungen bei 37°C für eine Stunde inkubiert. Die Träger werden dann entnommen und dreimal mit physiologischem Puffer gewaschen. Anschließend werden die Träger jeweils in eine Vertiefung einer Mikrotiterplatte gelegt, welche mit 200 µl eines Minimalmediums gefüllt ist. Die Träger werden für 24 Stunden bei 37°C inkubiert. Anschließend werden die Träger entnommen und verworfen. Zu jeder Vertiefung der Mikrotiterplatte werden 50 µl eines Vollmediums (Trypcase-soja, bioMerieux, Nr. 69280, Marcy l'Etoile, Frankreich) zugegeben. Anschließend wird die Trübung der Lösung im Abstand von 30 Minuten über einen Zeitraum von 48 Stunden gemessen. Die Lösung wird dabei auf einer Temperatur von 37°C gehalten. Die Trübungsmessung erfolgt mit Licht einer Wellenlänge von 578 nm mittels eines geeigneten Lesegeräts. Eine Trübung zeigt an, dass Bakterien von der Oberfläche des Trägers in die Umgebung abgegeben worden sind.

Zur Herstellung der Cremeproben wurde als Basiscreme "Cremaba Plus HT" der Firma Spinnrad^{®}, Certus Handels GmbH, 22848 Norderstedt, Deutschland, verwendet. Dabei handelt es sich um eine Emulsionsgrundlage mit folgenden Inhaltsstoffen: Aqua, Caprylic/Capric Triglyceride, Pentylene Glycol, Hydrogenated Lecithin, Butyrospermum Parkii, Glycerin, Squalane, Ceramide 3. In die Basiscreme ist folgender weiterer Bestandteil eingearbeitet worden:
Silikonöl mit einem Silber-Gehalt von 0,65% w/w; das Silber liegt darin in Form von Partikeln mit einem mittleren Durchmesser von 10 nm vor; das Silber wird im Folgenden als "nanodisperses Silber" bezeichnet;
   oder
in Pulverform vorliegende Agglomerate metallischen Silbers mit einer mittleren Porosität von 80% und einem mittleren Durchmesser von 5 µm; das Silber wird im Folgenden als "Agglomerat-Silber" bezeichnet.

Es wurden Cremes mit 0,01% w/w nanodispersem Silber sowie mit 0,1% w/w und 0,5% w/w Agglomerat-Silber hergestellt. Darüber hinaus wurde eine Creme mit 0,05% w/w nanodispersem Silber hergestellt, wobei das nanodisperse Silber hier aus einer Legierung, bestehend aus 99,5% w/w Silber, 0,49% w/w Zink und 0,01% w/w Kupfer, bestand. Weiterhin wurde eine Creme mit 1,5% w/w Agglomerat-Silber hergestellt, wobei das Agglomerat-Silber hier aus einer Legierung, bestehend aus 99,5% w/w Silber, 0,49% w/w Zink und 0,01% w/w Kupfer, bestand.

Zur Herstellung der Cremes wurden die Substanzen jeweils in einem 50 ml Becherglas vermischt, in einem Wasserbad auf 75°C für 20 Minuten erwärmt und dann mittels eines Ultraturrax (Janke und Kunkel, Antrieb T25, Statordurchmesser 25 mm, Rotordurchmesser 17 mm) für 5 Minuten dispergiert. Anschließend wurde die Creme abgekühlt und nochmals durchmischt.

In Fig. 2 zeigt jedes Feld eine x-y-Grafik bei der auf der x-Achse die Zeit und auf der y-Achse die optische Dichte aufgetragen ist. Die in den Spalten 1 bis 8 der Fig. 2 dargestellten Versuchsergebnisse sind in parallelen, den Reihen A bis H entsprechenden Versuchsansätzen A bis H mit folgenden Cremes ermittelt worden:
- Spalte 1, Reihen A-H:: Creme ohne Silber-Zusätze
- Spalte 2, Reihen A-H:: Creme mit 0,1% w/w Agglomerat-Silber
- Spalte 3, Reihen A-H:: Creme mit 0,5% w/w Agglomerat-Silber
- Spalte 4, Reihen A-H:: Creme mit 1,5% w/w Agglomerat-Silber, bestehend aus 99,5% w/w Silber, 0,49% w/w Zink und 0,01% w/w Kupfer
- Spalte 5, Reihen A-H:: Creme mit 0,01% w/w nanodispersem Silber
- Spalte 6, Reihen A-H:: Creme mit 0,05% w/w nanodispersem Silber bestehend aus 99,5% w/w Silber, 0,49% w/w Zink und 0,01% w/w Kupfer
- Spalte 7, Reihe A:: Positivkontrolle
- Spalte 7, Reihe B:: Negativkontrolle
- Spalte 7, Reihe C:: Leerwert
- Spalte 8, Reihen A-H:: Sterilkontrollen

Bei der Positivkontrolle wurde ein metallisches Silber enthaltendes Polymer eingesetzt. Die Werte zeigen, dass die eingesetzten Bakterien gegenüber Silber sensitiv sind und davon abgetötet werden können. Bei der Negativkontrolle wurde das gleiche Polymer eingesetzt, dass jedoch kein Silber enthielt. Beim Leerwert handelt es sich um einen in einer leeren Vertiefung der Mikrotiterplatte gemessenen Wert, der bei der Auswertung von allen Messwerten subtrahiert wurde. Bei den Sterilkontrollen wurde jeweils nur Medium ohne Zusatz von Staphylococcus epidermidis eingesetzt, um zu zeigen, dass das Bakterienwachstum nicht vom Medium herrührt.

Die Ergebnisse lassen sich wie folgt zusammenfassen:

| Probenbezeichnung | Onset-OD [h] brutto | Onset-OD[h] netto | Wirkung |
|---|---|---|---|
| 1A-H Creme ohne Silber-Zusätze | 5,2 | 0 | **nicht antibakteriell** |
| 2A-H Creme mit 0,1% w/w Agglomerat-Silber | 18,4 | 13,2 | **hoch antibakteriell** |
| 3A-H Creme mit 0,5% w/w Agglomerat-Silber | 32,2 | 27,0 | **hoch antibakteriell** |
| 4A-H Creme mit 1,5% w/w Agglomerat-Silber, bestehend aus 99,5% w/w Silber, 0,49% w/w Zink und 0,01% w/w Kupfer | 37,9 | 32,7 | **hoch antibakteriell** |
| 5A-H Creme mit 0,01% w/w nanodispersem Silber | 35,3 | 30,1 | **hoch antibakteriell** |
| 6A-H Creme mit 0,05% w/w nanodispersem Silber, bestehend aus 99,5% w/w Silber, 0,49% w/w Zink und 0,01% w/w Kupfer | Limit | >42,8 | **bakterizid** |
| 7A/B Positivkontrolle/Negativkontrolle | Limit / 9,2 | - | **OK** |
| 8A-H Sterilkontrollen | Limit | - | **OK** |
| 7C Leerwert | | - | **OK** |

"Onset-OD [h] brutto" bezeichnet die in Stunden bemessene Zeit, bis es zu einem exponenziellen Anstieg der optischen Dichte (OD) um 0,2 kam. "Onset-OD [h] netto" ergibt sich aus "Onset-OD [h] brutto" durch jeweiligen Abzug des für die Creme ohne Silber-Zusätze ermittelten Werts "Onset-OD [h] brutto". Bei parallelen Versuchsansätzen ist jeweils der Mittelwert angegeben. "Antibakteriell" bezeichnet eine Wirkung, bei der das Wachstum der Bakterien verzögert wird, während "bakterizid" eine Wirkung bezeichnet, bei der die Bakterien zu 100% abgetötet werden, so dass kein Bakterienwachstum mehr beobachtet werden kann.

Die Versuchsergebnisse zeigen, dass Agglomerat-Silber wie nanodisperses Silber hoch antibakteriell wirkt. Nanodisperses Silber ist bei geringeren Silberkonzentrationen wirksam als Agglomerat-Silber. Mit Agglomerat-Silber lässt sich jedoch noch immer eine hoch antibakterielle Wirkung erzielen. Sowohl die Wirkung des Agglomerat-Silbers als auch die Wirkung des nanodispersen Silbers ist in den Cremes gesteigert, die neben Silber zusätzlich Zink und Kupfer enthalten.

## Patentansprüche

1. Körperpflegemittel, enthaltend metallische Partikel, von denen im Körperpflegemittel oder bei einem Kontakt mit Körperflüssigkeit oder Körperfeuchtigkeit Zink-Ionen und Silber-Ionen freigesetzt werden, wobei die Partikel einen Gehalt an metallischem Silber von mindestens 99% w/w aufweisen.

2. Körperpflegemittel nach Anspruch 1, wobei von den Partikeln in einer definierten Zeiteinheit mehr Silber-Ionen als Zink-Ionen freigesetzt werden.

3. Körperpflegemittel nach einem der vorhergehenden Ansprüche, wobei von den Partikeln im Körperpflegemittel oder bei dem Kontakt mit der Körperflüssigkeit oder Körperfeuchtigkeit weiterhin Kupfer-Ionen freigesetzt werden.

4. Körperpflegemittel nach Anspruch 3, wobei von den Partikeln in der Zeiteinheit mehr Silber-Ionen als Kupfer-Ionen freigesetzt werden.

5. Körperpflegemittel nach einem der vorhergehenden Ansprüche, wobei die Partikel einen Gehalt an metallischem Silber von mindestens 99,5% w/w aufweisen.

6. Körperpflegemittel nach einem der vorhergehenden Ansprüche, wobei die Partikel bis zu 0,5% w/w metallisches Zink und bis zu 0,5% w/w metallisches Kupfer enthalten.

7. Körperpflegemittel nach einem der vorhergehenden Ansprüche, wobei die Partikel aus einer Silber-Zink-Legierung oder einer Silber-Zink-Kupfer-Legierung gebildet sind.

8. Körperpflegemittel nach einem der vorhergehenden Ansprüche, wobei die Partikel Verunreinigungen von weniger als 5 ppm an Kalium, Natrium oder Chlor aufweisen.

9. Körperpflegemittel nach einem der vorhergehenden Ansprüche, wobei die darin enthaltenen Partikel einen Durchmesser von 1 bis 50 nm, insbesondere 5 bis 15 nm, vorzugsweise 10 nm, aufweisen.

10. Körperpflegemittel nach einem der vorhergehenden Ansprüche, wobei das Körperpflegemittel keine Hilfsmittel zum Dispergieren der Partikel enthält.

11. Körperpflegemittel nach einem der vorhergehenden Ansprüche, wobei das Körperpflegemittel neben den Partikeln keine Konservierungsstoffe enthält.

12. Körperpflegemittel nach einem der vorhergehenden Ansprüche, wobei zumindest ein Teil der Partikel metallisches Silber enthaltende poröse Partikel mit einem mittleren Durchmesser zwischen 1 und 100 µm sind.

13. Körperpflegemittel nach Anspruch 12, wobei die Partikel eine mittlere innere Porosität von mindestens 65%, insbesondere zwischen 65 und 95%, bevorzugt zwischen 65 und 90%, insbesondere zwischen 70 und 85%, vorzugsweise zwischen 75 und 85%, oder bevorzugt zwischen 85 und 95%, insbesondere zwischen 90 und 95%, aufweisen.

14. Körperpflegemittel nach Anspruch 12 oder 13, wobei die Partikel als Agglomerate metallischer Primärpartikel vorliegen.

15. Körperpflegemittel nach Anspruch 14, wobei die Primärpartikel einen mittleren Durchmesser zwischen 10 und 200 nm, vorzugsweise zwischen 15 und 80 nm, aufweisen.

16. Körperpflegemittel nach Anspruch 14 oder 15, wobei der mittlere Abstand zwischen den jeweils äußersten Primärpartikeln an der Oberfläche der Agglomerate im Bereich von 20 bis 200 nm, vorzugsweise 100 bis 200 nm, liegt.

17. Körperpflegemittel nach einem der Ansprüche 12 bis 16, wobei die Partikel eine schwammartige Struktur aufweisen.

18. Körperpflegemittel nach einem der Ansprüche 12 bis 17, wobei die Partikel einen mittleren Außendurchmesser von 2 bis 20 µm, vorzugsweise 2 bis 5 µm, aufweisen.

19. Körperpflegemittel nach einem der Ansprüche 12 bis 18, wobei die Partikel eine spezifische Oberfläche zwischen 2 und 10 m²/g, insbesondere zwischen 3 und 6 m²/g, vorzugsweise zwischen 3,5 und 4,5 m²/g, aufweisen.

20. Körperpflegemittel nach einem der vorhergehenden Ansprüche, wobei darin die Partikel in einem Trägermaterial enthalten sind, welches aus einem Silikonöl, einem Mineralöl, Glyzerin oder einem Salbenbestandteil besteht.

21. Körperpflegemittel nach einem der vorhergehenden Ansprüche, wobei das Körperpflegemittel ein, insbesondere medizinisch wirksames, Präparat, wie eine Emulsion, eine Lotion, ein Gel, eine Creme, eine Salbe, eine Heilsalbe, ein Puder, ein Kosmetikum, eine Hautschutzcreme oder -salbe, ein Desinfektionsmittel, eine Suspension, eine Seife, ein synthetisches Tensid, ein Badezusatz, ein Peelingpräparat, ein Gesichtswasser, ein Zahnpflegemittel, eine Zahncreme, ein Mundwasser, ein Zahnreinigungskaugummi, ein Prothesenhaftmittel, ein Haarshampoo oder ein Sonnenschutzmittel ist.

22. Verfahren zur Herstellung eines Körperpflegemittels nach einem der Ansprüche 1 bis 21, wobei die Partikel durch einen Sputterprozess hergestellt werden, bei dem das die Partikel bildende Metall verdampft wird und der metallische Dampf direkt in eine Trägerflüssigkeit abgeschieden wird, die dann in das Körperpflegemittel eingebracht wird.

23. Verfahren nach Anspruch 22, wobei die Trägerflüssigkeit ein Silikonöl, ein Mineralöl, Glyzerin oder ein Salbenbestandteil ist.

24. Verfahren nach Anspruch 22 oder 23, wobei die Trägerflüssigkeit bei 20 °C einen Dampfdruck von weniger als 250 mbar, insbesondere weniger als 70 mbar, vorzugsweise weniger als 10 mbar, insbesondere weniger als 3 mbar, aufweist.

25. Verwendung von metallischen Partikeln zur Herstellung eines Medikaments zur topischen Behandlung einer Entzündung und/oder Infektion bei einem Säugetier oder Menschen, wobei von den Partikeln im Medikament oder bei einem Kontakt mit Körperflüssigkeit oder Körperfeuchtigkeit Zink-Ionen und Silber-Ionen freigesetzt werden, wobei die Partikel einen Gehalt an metallischem Silber von mindestens 99% w/w aufweisen.

26. Verwendung nach Anspruch 25, wobei das Medikament eine Salbe, eine Creme oder ein Gel ist.

27. Verwendung nach Anspruch 25 oder 26, wobei von den Partikeln in einer definierten Zeiteinheit mehr Silber-Ionen als Zink-Ionen freigesetzt werden.

28. Verwendung nach einem der Ansprüche 25 bis 27, wobei von den Partikeln im Medikament oder bei einem Kontakt mit Körperflüssigkeit oder Körperfeuchtigkeit weiterhin Kupfer-Ionen freigesetzt werden.

29. Verwendung nach einem der Ansprüche 25 bis 28, wobei von den Partikeln in der Zeiteinheit mehr Silber-Ionen als Kupfer-Ionen freigesetzt werden.

30. Verwendung nach einem der Ansprüche 25 bis 29, wobei die Partikel einen Gehalt an metallischem Silber von mindestens 99,5% w/w aufweisen.

31. Verwendung nach einem der Ansprüche 25 bis 30, wobei die Partikel bis zu 0,5% w/w metallisches Zink und bis zu 0,5% w/w metallisches Kupfer enthalten.

32. Verwendung nach einem der Ansprüche 25 bis 31, wobei die Partikel aus einer Silber-Zink-Legierung oder einer Silber-Zink-Kupfer-Legierung gebildet sind.

33. Verwendung nach einem der Ansprüche 25 bis 32, wobei die Partikel Verunreinigungen von weniger als 5 ppm an Kalium, Natrium oder Chlor aufweisen.

34. Verwendung nach einem der Ansprüche 25 bis 33, wobei die in dem Medikament enthaltenen Partikel einen Durchmesser von 1 bis 50 nm, insbesondere 5 bis 15 nm, vorzugsweise 10 nm, aufweisen.

35. Verwendung nach einem der Ansprüche 25 bis 34, wobei das Medikament keine Hilfsmittel zum Dispergieren der Partikel enthält.

36. Verwendung nach einem der Ansprüche 25 bis 35, wobei das Medikament neben den Partikeln keine Konservierungsstoffe enthält.

37. Verwendung nach einem der Ansprüche 25 bis 36, wobei zumindest ein Teil der Partikel metallisches Silber enthaltende poröse Partikel mit einem mittleren Durchmesser zwischen 1 und 100 µm sind.

38. Verwendung nach Anspruch 37, wobei die mittlere innere Porosität der Partikel mindestens 65%, insbesondere zwischen 65 und 95%, bevorzugt zwischen 65 und 90%, insbesondere zwischen 70 und 85%, vorzugsweise zwischen 75 und 85%, oder bevorzugt zwischen 85 und 95%, insbesondere zwischen 90 und 95%, beträgt.

39. Verwendung nach Anspruch 37 oder 38, wobei die Partikel als Agglomerate metallischer Primärpartikel vorliegen.

40. Verwendung nach Anspruch 39, wobei die Primärpartikel einen mittleren Durchmesser zwischen 10 und 200 nm, vorzugsweise zwischen 15 und 80 nm, aufweisen.

41. Verwendung nach Anspruch 39 oder 40, wobei der mittlere Abstand zwischen den jeweils äußersten Primärpartikeln an der Oberfläche der Agglomerate im Bereich von 20 bis 200 nm, vorzugsweise 100 bis 200 nm, liegt.

42. Verwendung nach einem der Ansprüche 25 bis 41, wobei die Partikel eine schwammartige Struktur aufweisen.

43. Verwendung nach einem der Ansprüche 25 bis 42, wobei die Partikel einen mittleren Außendurchmesser von 2 bis 20 µm, vorzugsweise 2 bis 5 µm, aufweisen.

44. Verwendung nach einem der Ansprüche 25 bis 43, wobei die Partikel eine spezifische Oberfläche zwischen 2 und 10 m²/g, insbesondere zwischen 3 und 6 m²/g, vorzugsweise zwischen 3,5 und 4,5 m²/g, aufweisen.

45. Verwendung nach einem der Ansprüche 25 bis 44, wobei in dem Medikament die Partikel in einem Trägermaterial enthalten sind, welches aus einem Silikonöl, einem Mineralöl, Glyzerin oder einem Salbenbestandteil besteht.

## Claims

1. A body care composition containing metallic particles from which zinc ions and silver ions are released in the body care composition or on contact with body fluid or body moistness, the particles having a content of metallic silver of at least 99% w/w.

2. The body care composition as claimed in claim 1, more silver ions than zinc ions being released from the particles in a defined time unit.

3. The body care composition as claimed in one of the preceding claims, copper ions furthermore being released from the particles in the body care composition or on contact with the body fluid or body moistness.

4. The body care composition as claimed in claim 3, more silver ions than copper ions being released from the particles in the time unit.

5. The body care composition as claimed in one of the preceding claims, the particles having a content of metallic silver of at least 99.5% w/w.

6. The body care composition as claimed in one of the preceding claims, the particles containing up to 0.5% w/w of metallic zinc and up to 0.5% w/w of metallic copper.

7. The body care composition as claimed in one of the preceding claims, the particles being formed from a silver-zinc alloy or a silver-zinc-copper alloy.

8. The body care composition as claimed in one of the preceding claims, the particles containing impurities of less than 5 ppm of potassium, sodium or chlorine.

9. The body care composition as claimed in one of the preceding claims, the particles contained therein having a diameter of 1 to 50 nm, in particular 5 to 15 nm, preferably 10 nm.

10. The body care composition as claimed in one of the preceding claims, the body care composition containing no excipients for dispersing the particles.

11. The body care composition as claimed in one of the preceding claims, the body care composition containing no preservatives in addition to the particles.

12. The body care composition as claimed in one of the preceding claims, at least a part of the particles being porous particles containing metallic silver having a mean diameter of between 1 and 100 µm.

13. The body care composition as claimed in claim 12, the particles having a mean inner porosity of at least 65%, in particular between 65 and 95%, preferably between 65 and 90%, in particular between 70 and 85%, preferably between 75 and 85%, or preferably between 85 and 95%, in particular between 90 and 95%.

14. The body care composition as claimed in claim 12 or 13, the particles being present as agglomerates of metallic primary particles.

15. The body care composition as claimed in claim 14, the primary particles having a mean diameter of between 10 and 200 nm, preferably between 15 and 80 nm.

16. The body care composition as claimed in claim 14 or 15, the mean distance between the outermost primary particles on the surface of the agglomerates in each case lying in the range from 20 to 200 nm, preferably 100 to 200 nm.

17. The body care composition as claimed in one of claims 12 to 16, the particles having a spongy structure.

18. The body care composition as claimed in one of claims 12 to 17, the particles having a mean outer diameter of 2 to 20 µm, preferably 2 to 5 µm.

19. The body care composition as claimed in one of claims 12 to 18, the particles having a specific surface area of between 2 and 10 m²/g, in particular between 3 and 6 m²/g, preferably between 3.5 and 4.5 m²/g.

20. The body care composition as claimed in one of the preceding claims, the particles therein being contained in a carrier material which consists of a silicone oil, a mineral oil, glycerol or an ointment constituent.

21. The body care composition as claimed in one of the preceding claims, the body care composition being a, in particular medicinally active, preparation, such as an emulsion, a lotion, a gel, a cream, an ointment, a healing ointment, a powder, a cosmetic, a skin protection cream or ointment, a disinfectant, a suspension, a soap, a synthetic surfactant, a bath additive, a peeling preparation, a face lotion, a dental care composition, a toothpaste, a mouthwash, a tooth-cleaning chewing gum, a prosthesis adhesive, a hair shampoo or a sunscreen composition.

22. A process for the production of a body care composition as claimed in one of claims 1 to 21, the particles being produced by a sputtering process in which the metal forming the particles is evaporated and the metallic vapor is deposited directly in a carrier liquid which is then incorporated into the body care composition.

23. The process as claimed in claim 22, the carrier liquid being a silicone oil, a mineral oil, glycerol or an ointment constituent.

24. The process as claimed in claim 22 or 23, the carrier liquid at 20° C having a vapor pressure of less than 250 mbar, in particular less than 70 mbar, preferably less than 10 mbar, in particular less than 3 mbar.

25. The use of metallic particles for production of a medicament for the topical treatment of an inflammation and/or infection in a mammal or human, zinc ions and silver ions being released from the particles in the medicament or on contact with body fluid or body moistness, the particles having a content of metallic silver of at least 99% w/w.

26. The use as claimed in claim 25, the medicament being an ointment, a cream or a gel.

27. The use as claimed in claim 25 or 26, more silver ions than zinc ions being released from the particles in a defined time unit.

28. The use as claimed in one of claims 25 to 27, copper ions furthermore being released from the particles in the medicament or on contact with body fluid or body moistness.

29. The use as claimed in one of claims 25 to 28, more silver ions than copper ions being released from the particles in the time unit.

30. The use as claimed in one of claims 25 to 29, the particles having a content of metallic silver of at least 99.5% w/w.

31. The use as claimed in one of claims 25 to 30, the particles containing up to 0.5% w/w of metallic zinc and up to 0.5% w/w of metallic copper.

32. The use as claimed in one of claims 25 to 31, the particles being formed from a silver-zinc alloy or a silver-zinc-copper alloy.

33. The use as claimed in one of claims 25 to 32, the particles containing impurities of less than 5 ppm of potassium, sodium or chlorine.

34. The use as claimed in one of claims 25 to 33, the particles contained in the medicament having a diameter of 1 to 50 nm, in particular 5 to 15 nm, preferably 10 nm.

35. The use as claimed in one of claims 25 to 34, the medicament containing no excipients for dispersing the particles.

36. The use as claimed in one of claims 25 to 35, the medicament containing no preservatives in addition to the particles.

37. The use as claimed in one of claims 25 to 36, at least a part of the particles being porous particles containing metallic silver having a mean diameter of between 1 and 100 µm.

38. The use as claimed in claim 37, the mean inner porosity of the particles being at least 65%, in particular between 65 and 95%, preferably between 65 and 90%, in particular between 70 and 85%, preferably between 75 and 85%, or preferably between 85 and 95%, in particular between 90 and 95%.

39. The use as claimed in claim 37 or 38, the particles being present as agglomerates of metallic primary particles.

40. The use as claimed in claim 39, the primary particles having a mean diameter of between 10 and 200 nm, preferably between 15 and 80 nm.

41. The use as claimed in claim 39 or 40, the mean distance between the outermost primary particles on the surface of the agglomerates in each case lying in the range from 20 to 200 nm, preferably 100 to 200 nm.

42. The use as claimed in one of claims 25 to 41, the particles having a spongy structure.

43. The use as claimed in one of claims 25 to 42, the particles having a mean outer diameter of 2 to 20 µm, preferably 2 to 5 µm.

44. The use as claimed in one of claims 25 to 43, the particles having a specific surface area of between 2 and 10 m²/g, in particular between 3 and 6 m²/g, preferably between 3.5 and 4.5 m²/g.

45. The use as claimed in one of claims 25 to 44, the particles in the medicament being contained in a carrier material which consists of a silicone oil, a mineral oil, glycerol or an ointment constituent.

## Revendications

1. Produit de soin du corps contenant des particules métalliques depuis lesquelles des ions zinc et des ions argent sont libérés dans le produit de soin du corps ou lors d'un contact avec un fluide corporel ou l'humidité corporelle, dans lequel les particules présentent une teneur en argent métallique au moins égale à 99 % en masse.

2. Produit de soin du corps selon la revendication 1, dans lequel, sur une unité de temps définie, plus d'ions argent que d'ions zinc sont libérés par les particules.

3. Produit de soin du corps selon l'une des revendications précédentes, dans lequel des ions cuivre sont en outre libérés par les particules dans le produit de soin du corps ou lors du contact avec un fluide corporel ou l'humidité corporelle.

4. Produit de soin du corps selon la revendication 3, dans lequel plus d'ions argent que d'ions cuivre sont libérés par les particules sur l'unité de temps.

5. Produit de soin du corps selon l'une des revendications précédentes, dans lequel les particules présentent une teneur en argent métallique au moins égale à 99,5 % en masse.

6. Produit de soin du corps selon l'une des revendications précédentes, dans lequel les particules contiennent jusqu'à 0,5 % en masse de zinc métallique et jusqu'à 0,5 % en masse de cuivre métallique.

7. Produit de soin du corps selon l'une des revendications précédentes, dans lequel les particules sont formées d'un alliage d'argent - zinc ou d'un alliage d'argent - zinc - cuivre.

8. Produit de soin du corps selon l'une des revendications précédentes, dans lequel les particules présentent des impuretés, à raison de moins de 5 ppm, de potassium, sodium ou chlore.

9. Produit de soin du corps selon l'une des revendications précédentes, dans lequel les particules qu'il contient présentent un diamètre compris entre 1 et 50 nm, en particulier entre 5 et 15 nm, de préférence égal à 10 nm.

10. Produit de soin du corps selon l'une des revendications précédentes, dans lequel le produit de soin du corps ne contient aucun adjuvant d'aide à la dispersion des particules.

11. Produit de soin du corps selon l'une des revendications précédentes, dans lequel le produit de soin du corps, outre les particules, ne contient aucun conservateur.

12. Produit de soin du corps selon l'une des revendications précédentes, dans lequel au moins une partie des particules sont des particules poreuses contenant de l'argent métallique présentant un diamètre moyen compris entre 1 et 100 µm.

13. Produit de soin du corps selon la revendication 12, dans lequel les particules présentent une porosité interne moyenne d'au moins 65 %, en particulier comprise entre 65 et 95 %, de préférence entre 65 et 90 %, en particulier entre 70 et 85 %, de préférence entre 75 et 85 %, ou de préférence entre 85 et 95 %, en particulier entre 90 et 95 %.

14. Produit de soin du corps selon la revendication 12 ou 13, dans lequel les particules se présentent sous forme d'agglomérats de particules primaires métalliques.

15. Produit de soin du corps selon la revendication 14, dans lequel les particules primaires présentent un diamètre moyen compris entre 10 et 200 nm, de préférence entre 15 et 80 nm.

16. Produit de soin du corps selon la revendication 14 ou 15, dans lequel l'écart moyen entre les particules primaires respectivement les plus externes à la surface des agglomérats est compris dans la gamme allant de 20 à 200 nm, de préférence de 100 à 200 nm.

17. Produit de soin du corps selon l'une des revendications 12 à 16, dans lequel les particules présentent une structure de type éponge.

18. Produit de soin du corps selon l'une des revendications 12 à 17, dans lequel les particules présentent un diamètre externe moyen compris entre 2 et 20 µm, de préférence entre 2 et 5 µm.

19. Produit de soin du corps selon l'une des revendications 12 à 18, dans lequel les particules présentent une surface spécifique comprise entre 2 et 10 m²/g, en particulier entre 3 et 6 m²/g, de préférence entre 3,5 et 4,5 m²/g.

20. Produit de soin du corps selon l'une des revendications précédentes, dans lequel, à l'intérieur, les particules sont contenues dans un matériau de support qui se compose d'une huile silicone, d'une huile minérale, de glycérine ou d'un ingrédient de pommade.

21. Produit de soin du corps selon l'une des revendications précédentes, dans lequel le produit de soin du corps est une préparation, en particulier efficace sur le plan médical, comme une émulsion, une lotion, un gel, une crème, une pommade, un onguent, une poudre, un produit cosmétique, une crème ou une pommade protectrice de la peau, un agent désinfectant, une suspension, un savon, un tensioactif synthétique, un adjuvant de bain, une préparation pour peeling ou gommage, une lotion pour visage, un produit de soins dentaires, une pâte dentifrice, une solution pour bain de bouche, un chewing-gum pour le nettoyage dentaire, un fixatif pour prothèse, un shampoing capillaire ou un écran solaire.

22. Procédé de fabrication d'un produit de soin du corps selon l'une des revendications 1 à 21, dans lequel les particules sont fabriquées par un processus de pulvérisation, dans lequel le métal formant les particules est vaporisé et la vapeur métallique est directement déposée dans un fluide de support qui est ensuite introduit dans le produit de soin du corps.

23. Procédé selon la revendication 22, dans lequel le fluide de support est une huile de silicone, une huile minérale, de la glycérine ou un ingrédient de pommade.

24. Procédé selon la revendication 22 ou 23, dans lequel le fluide de support présente à 20 °C une pression de vapeur inférieure à 250 mbar, en particulier inférieure à 70 mbar, de préférence inférieure à 10 mbar, en particulier inférieure à 3 mbar.

25. Utilisation de particules métalliques pour fabriquer un médicament destiné au traitement topique d'une inflammation et/ou d'une infection chez un mammifère ou chez l'homme, dans laquelle des ions zinc et des ions argent sont libérés par les particules dans le médicament ou lors d'un contact avec un fluide corporel ou l'humidité corporelle, les particules présentant une teneur en argent métallique au moins égale à 99 % en masse.

26. Utilisation selon la revendication 25, dans laquelle le médicament est une pommade, une crème ou un gel.

27. Utilisation selon la revendication 25 ou 26, dans laquelle, sur une unité de temps définie, plus d'ions argent que d'ions zinc sont libérés par les particules.

28. Utilisation selon l'une des revendications 25 à 27, dans laquelle des ions cuivre sont en outre libérés dans le médicament par les particules ou lors d'un contact avec du fluide corporel ou l'humidité corporelle.

29. Utilisation selon l'une des revendications 25 à 28, dans laquelle plus d'ions argent que d'ions cuivre sont libérés par les particules sur l'unité de temps.

30. Utilisation selon l'une des revendications 25 à 29, dans laquelle les particules présentent une teneur en argent métallique au moins égale à 99,5 % en masse.

31. Utilisation selon l'une des revendications 25 à 30, dans laquelle les particules contiennent jusqu'à 0,5 % en masse de zinc métallique et jusqu'à 0,5 % en masse de cuivre métallique.

32. Utilisation selon l'une des revendications 25 à 31, dans laquelle les particules sont formées d'un alliage d'argent - zinc ou d'un alliage d'argent - zinc - cuivre.

33. Utilisation selon l'une des revendications 25 à 32, dans laquelle les particules présentent des impuretés, à raison de moins de 5 ppm, de potassium, sodium ou chlore.

34. Utilisation selon l'une des revendications 25 à 33, dans laquelle les particules contenues dans le médicament présentent un diamètre compris entre 1 et 50 nm, en particulier entre 5 et 15 nm, de préférence égal à 10 nm.

35. Utilisation selon l'une des revendications 25 à 34, dans laquelle le médicament ne contient aucun adjuvant d'aide à la dispersion des particules.

36. Utilisation selon l'une des revendications 25 à 35, dans laquelle le médicament, outre les particules, ne contient aucun conservateur.

37. Utilisation selon l'une des revendications 25 à 36, dans laquelle au moins une partie des particules sont des particules poreuses contenant de l'argent métallique présentant un diamètre moyen compris entre 1 et 100 µm.

38. Utilisation selon la revendication 37, dans laquelle la porosité interne moyenne des particules est d'au moins 65 %, en particulier comprise entre 65 et 95 %, de préférence entre 65 et 90 %, en particulier entre 70 et 85 %, de préférence entre 75 et 85 %, ou de préférence entre 85 et 95 %, en particulier entre 90 et 95 %.

39. Utilisation selon la revendication 37 ou 38, dans laquelle les particules se présentent sous forme d'agglomérats de particules primaires métalliques.

40. Utilisation selon la revendication 39, dans laquelle les particules primaires présentent un diamètre moyen compris entre 10 et 200 nm, de préférence entre 15 et 80 nm.

41. Utilisation selon la revendication 39 ou 40, dans laquelle l'écart moyen entre les particules primaires respectivement les plus externes à la surface des agglomérats est compris dans la gamme allant de 20 à 200 nm, de préférence de 100 à 200 nm.

42. Utilisation selon l'une des revendications 25 à 41, dans laquelle les particules présentent une structure de type éponge.

43. Utilisation selon l'une des revendications 25 à 42, dans laquelle les particules présentent un diamètre externe moyen compris entre 2 et 20 µm, de préférence entre 2 et 5 µm.

44. Utilisation selon l'une des revendications 25 à 43, dans laquelle les particules présentent une surface spécifique comprise entre 2 et 10 m²/g, en particulier entre 3 et 6 m²/g, de préférence entre 3,5 et 4,5 m²/g.

45. Utilisation selon l'une des revendications 25 à 44, dans laquelle, dans le médicament, les particules sont contenues dans un matériau de support qui se compose d'une huile de silicone, d'une huile minérale, de glycérine ou d'un ingrédient de pommade.
